# EUROPEAN PATENT APPLICATION

(11) **EP 4 822 644 A1**
(43) Date of publication of application: **14.10.2026**
(21) Application number: 25169756.1
(22) Date of filing: 10.04.2025
(51) Int. Cl.: G06T 7/33, G06T 7/00, A61B 6/00, A61B 6/50

(54) **RIGID-MOTION COMPENSATION FOR CORONARY ANGIOGRAMS**

(71) Applicant: Siemens Healthineers AG, 91301 Forchheim (DE)
(72) Inventor: CIMEN, Serkan, West Orange, 07052 (US)
(74) Representative: Kraus & Lederer PartGmbB

(57) **Abstract**

The invention relates to processing multiple cardiac images depicting a portion of coronary arteries within an anatomical region of interest. A respective set of lumen radius measurements is determined based on each of the multiple cardiac images and comprises multiple lumen radius measurements respectively associated with multiple locations of the portion of the coronary arteries. One or more locations of the multiple locations, at each of which at least two sets of the respective sets of lumen radius measurements all have a local or relative extremum, e.g., either a local maximum or a local minimum, are determined based on the respective sets of lumen radius measurements. Rigid-motion artifacts in the multiple cardiac images are corrected based on the one or more locations.

## Description

### TECHNICAL FIELD

Various examples of the disclosure generally relate to angiography. Various examples specifically relate to processing multiple cardiac images depicting a portion of coronary arteries within an anatomical region of interest to determine one or more vessel landmarks for correcting rigid-motion artifacts in the multiple cardiac images.

### BACKGROUND

3D reconstruction of coronary arteries from 2D X-ray angiograms is crucial for diagnosing cardiovascular diseases, guiding interventions, and improving surgical planning. Since angiograms often provide 2D projections of a 3D structure, advanced computational techniques are used to recover the 3D geometry.

In order to reconstruct 3D coronary arteries, at least two angiographic projections from different angles are acquired using such as an X-ray imaging scanner. However, X-ray imaging scanners normally generate non-simultaneous angiographic projections, which are prone to several rigid and/or non-rigid motion artifacts (or misalignments), including mainly patient or imaging device related movements, respiratory motion, and cardiac motion. To achieve an accurate 3D reconstruction of coronary arteries, these artifacts or misalignments need to be compensated or corrected before reconstruction or jointly with the reconstruction.

### SUMMARY

Accordingly, there is a need for advanced techniques for correcting rigid and/or non-rigid motion artifacts in cardiac images or angiographic projections. Specifically, a need exists for advanced techniques of automatically and precisely correcting rigid-motion artifacts in multiple cardiac images acquired from different angles. A need exists for precisely and reliably reconstructing 3D coronary arteries for a specific patient.

This need is met by the features of independent claims. The features of the dependent claims define embodiments.

Hereinafter, techniques for correcting rigid-motion artifacts in multiple cardiac images depicting a portion of coronary arteries within an anatomical region of interest will be described. The corrected cardiac images can be used to reconstruct 3D coronary arteries for a patient.

A computer-implemented method is provided. The method comprises obtaining multiple cardiac images. Each of the multiple cardiac images depicts a portion of coronary arteries within an anatomical region of interest. The method further comprises determining, based on each of the multiple cardiac images, a respective set of lumen radius measurements comprising multiple lumen radius measurements respectively associated with multiple locations of the portion of the coronary arteries. The method also comprises determining, based on the respective sets of lumen radius measurements, one or more locations of the multiple locations at each of which at least two sets of the respective sets of lumen radius measurements all have a local extremum. The method still comprises correcting, based on the one or more locations, rigid-motion artifacts in the multiple cardiac images.

According to various examples, the multiple cardiac images may comprise cardiac images acquired under different acquisition angles.

A computing device comprising a processor and a memory is provided. Upon loading and executing program code from the memory, the processor is configured to perform a method. The method comprises obtaining multiple cardiac images. Each of the multiple cardiac images depicts a portion of coronary arteries within an anatomical region of interest. The method further comprises determining, based on each of the multiple cardiac images, a respective set of lumen radius measurements comprising multiple lumen radius measurements respectively associated with multiple locations of the portion of the coronary arteries. The method also comprises determining, based on the respective sets of lumen radius measurements, one or more locations of the multiple locations at each of which at least two sets of the respective sets of lumen radius measurements all have a local extremum. The method still comprises correcting, based on the one or more locations, rigid-motion artifacts in the multiple cardiac images.

An angiography device comprising a computing device is provided. The computing device comprises a processor and a memory. Upon loading and executing program code from the memory, the processor is configured to perform a method. The method comprises obtaining multiple cardiac images. Each of the multiple cardiac images depicts a portion of coronary arteries within an anatomical region of interest. The method further comprises determining, based on each of the multiple cardiac images, a respective set of lumen radius measurements comprising multiple lumen radius measurements respectively associated with multiple locations of the portion of the coronary arteries. The method also comprises determining, based on the respective sets of lumen radius measurements, one or more locations of the multiple locations at each of which at least two sets of the respective sets of lumen radius measurements all have a local extremum. The method still comprises correcting, based on the one or more locations, rigid-motion artifacts in the multiple cardiac images.

A computer program or a computer-program product or a computer-readable storage medium includes program code is provided. The program code can be loaded from a memory and executed by a processor. Upon loading and executing program code from the memory, the processor is configured to perform a method. The method comprises obtaining multiple cardiac images. Each of the multiple cardiac images depicts a portion of coronary arteries within an anatomical region of interest. The method further comprises determining, based on each of the multiple cardiac images, a respective set of lumen radius measurements comprising multiple lumen radius measurements respectively associated with multiple locations of the portion of the coronary arteries. The method also comprises determining, based on the respective sets of lumen radius measurements, one or more locations of the multiple locations at each of which at least two sets of the respective sets of lumen radius measurements all have a local extremum. The method still comprises correcting, based on the one or more locations, rigid-motion artifacts in the multiple cardiac images.

It is to be understood that the features mentioned above and those yet to be explained below may be used not only in the respective combinations indicated, but also in other combinations or in isolation without departing from the scope of the invention.

### BRIEF DESCRIPTION OF THE DRAWINGS

FIG. 1 schematically illustrates aspects with respect to a C-arm machine.
FIG. 2 schematically illustrates an exemplary 3D frontal view of a heart and of coronary arteries.
FIG. 3 is a flowchart of a method according to various examples.
FIG. 4 schematically illustrates multiple frames of an angiogram acquired at different time points.
FIG. 5 schematically illustrates one exemplary frame of an angiogram according to various examples.
FIG. 6 schematically illustrates various exemplary curves respectively depicting different sets of lumen radius measurements according to various examples.
FIG. 7 is a block diagram of a computing device according to various examples.

### DETAILED DESCRIPTION OF THE DRAWINGS

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Hereinafter, techniques for correcting rigid-motion artifacts in multiple cardiac images are described. The rigid-motion artifacts are corrected by processing the multiple cardiac images, each of which may depict the same portion of coronary arteries within an anatomical region of interest. For example, each of the multiple cardiac images may depict the same portion of either the left coronary artery or the right coronary artery of the same patient.

The multiple cardiac images may be obtained either during or after an angiography exam of the portion of coronary arteries within the anatomical region of interest. The processing may take place either during or after the angiography exam of the portion of coronary arteries, i.e., either real-time processing or post-processing is possible.

The multiple cardiac images, each of which depicts a portion of coronary arteries, i.e., the same portion of coronary arteries, within an anatomical region of interest, are obtained either during or after an angiography exam. A respective set of lumen radius measurements is determined based on each of the multiple cardiac images and comprises multiple lumen radius measurements respectively associated with multiple locations of the portion of the coronary arteries. One or more locations of the multiple locations, at each of which at least two sets of the respective sets of lumen radius measurements all have a local or relative extremum, e.g., either a local maximum or a local minimum, are determined based on the respective sets of lumen radius measurements. Rigid-motion artifacts in the multiple cardiac images are corrected based on the one or more locations.

According to various example, a count of the one or more locations is preferably 4 to 6.

According to various examples, the multiple cardiac images may be acquired during the same cardiac phase. For example, the multiple cardiac images may be acquired during the same cardiac phase of different cardiac cycles or at the same time point of different cardiac cycles. For example, the multiple cardiac images may be acquired at the end of the diastolic phase of different cardiac cycles. I.e., the multiple cardiac images encapsulate information associated with substantively the same cardiac motion, e.g., respective states of the atria, the ventricles, and the valves.

According to various examples, the multiple cardiac images may comprise cardiac images acquired under different acquisition angles.

By processing the multiple cardiac images using techniques disclosed herein, one or more rigid-motion artifacts in multiple cardiac images can be compensated or corrected. I.e., one or more rigid-motion artifacts can be precisely and reliably compensated or corrected based on one or more precisely selected or determined vessel landmarks. Then, 3D coronary arteries can be reconstructed based on the corrected multiple cardiac images.

In general, the cardiac cycle is the performance of the human heart from the beginning of one heartbeat to the beginning of the next. It consists of two periods: one during which the heart muscle relaxes and refills with blood, called diastole, following a period of robust contraction and pumping of blood, called systole. After emptying, the heart relaxes and expands to receive another influx of blood returning from the lungs and other systems of the body, before again contracting to pump blood to the lungs and those systems. A normally performing heart must be fully expanded before it can efficiently pump again. Assuming a healthy heart and a typical rate of 70 to 75 beats per minute, each cardiac cycle, or heartbeat, takes about 0.8 second to complete the cycle.

Events and details of cardiac cycles can be illustrated using a Wiggers diagram. In the Wiggers diagram, the X-axis is used to plot time subdivided into the cardiac phases, while the Y-axis typically contains the following on a single grid: blood pressure, aortic pressure, ventricular pressure, atrial pressure, ventricular volume, and electrocardiogram. Accordingly, a specific cardiac cycle can be determined using electrocardiography.

According to various examples, the multiple cardiac images may be obtained directly from an angiography device or from a database for storing the multiple cardiac images acquired by the angiography device, e.g., a picture archiving and communication system (PACS).

Generally, the typical maximum frame rates found on an angiography device such as a C-arm X-ray machine are 8, 15, or 30. This means that during a single cardiac cycle of 0.8 second, 6, 12, or 24 frames can be acquired by a C-arm X-ray machine.

According to various examples, the angiography device may comprise a C-arm X-ray machine, an intravascular ultrasound machine, an optical coherence tomography machine, or a coronary computed tomography angiography machine.

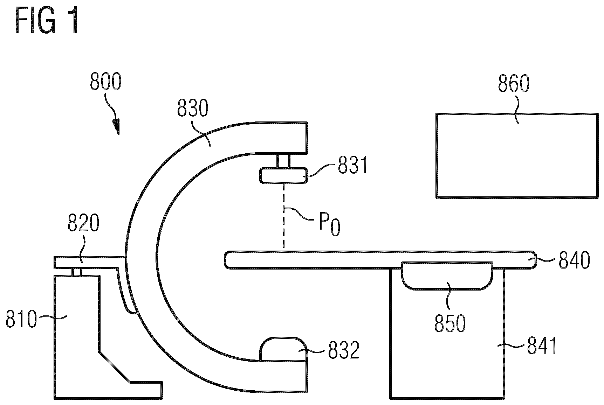

As an exemplary angiography device, FIG. 1 schematically illustrates aspects with respect to a C-arm machine 800. The C-arm machine 800 may be used for both angiography and fluoroscopy. In FIG. 1, the C-arm machine 800 is in neutral position P0. It is possible to manipulate the C-arm machine 800 to be in a rotated position deviating from the neutral position P0. The angle between the neutral position P0 and a specific rotated position is referred to as the angiography angle or the fluoroscopy angle. The C-arm machine 800 comprises a rotatable C arm 830 on which X-ray emission means 831 and X-ray detection means 832 may be mounted. The C arm 830 and thereby X-ray emission means 831 and X-ray detection means 832 are positioned to center around patient surface 840. X-ray emission means 831 may emit X-rays which may penetrate through a patient positioned on the patient surface 840. X-ray detection means 832 detects the X-rays emitted from X-ray emission means 831. When a patient on the patient surface 840 is injected with a radio-opaque contrast agent into the patient's vessels, some of the X-rays emitted by X-ray emission means 831 are absorbed by the radio-opaque contrast agent, leading X-ray detection means 832 to detect a sequence of images of the vessels filled with the radio-opaque contrast agent, i.e. an angiogram. X-ray emission means 831 and X-ray detection means 832 may also collectively be referred to as x-ray imaging means.

The C arm 830 may be coupled to a C arm rotation unit 820. The C arm rotation unit 820 may be any motorized means configured to rotate the C arm 830 according to an angiography angle or a fluoroscopy angle. The C arm rotation unit 820 may be attached to and controlled by a C arm control until 810. The C arm control unit 810 may be any kind of circuitry capable of controlling C arm 830. For example, the C arm control unit 810 may include a computing device.

The C-arm machine 800 may further include a control panel 850 mounted onto a side surface of patient surface support 841. The control panel 850 may be used to control C arm 830 in order to guide medical specialists to pathological vessels. Fig. 1 does not show any connections between control panel 850 and C arm 830 to simplify the depiction of the exemplary C-arm machine 800. In some examples, the connection may be wireless. In some further examples, the connection may be wired and may e.g., be integrated into the ceiling of the room where the C-arm machine 800 is located.

The C-arm machine 800 may also include a display 860. The display 860 may be used to display information to the medical specialist, such as real-time fluoroscopy image with an overlaid vessel roadmap image including a path to one or more pathological vessels, and/or a location of a catheter tip. Further, the display 860 may be used to display vessel segmentation data included in overlaid vessel roadmap images, including labels for various vessel segments. In some examples, display 860 may be a touch screen, which may be used to toggle the display of the vessel segmentation data on and off.

The C-arm machine 800 may be connectable to a database (not shown in FIG. 1), such as a PACS located within a local network of a hospital, for storing angiograms and/or lumen radius measurements respectively associated with multiple locations of a portion of coronary arteries.

According to various examples, a name (e.g., any one in the column "Abbreviation" in Table 1) of a specific segment of a coronary artery or of the entire coronary artery can be determined based on an anatomical region of interest of the angiography exam. For example, such an anatomical region of interest may comprise the left or right branch of the coronary vessel tree or a specific vessel section. The anatomical region of interest may be determined based on user input, or on a patient's electronic medical record included in a health information system. Additionally or optionally, the anatomical region of interest may be determined based on multiple predefined seed points.

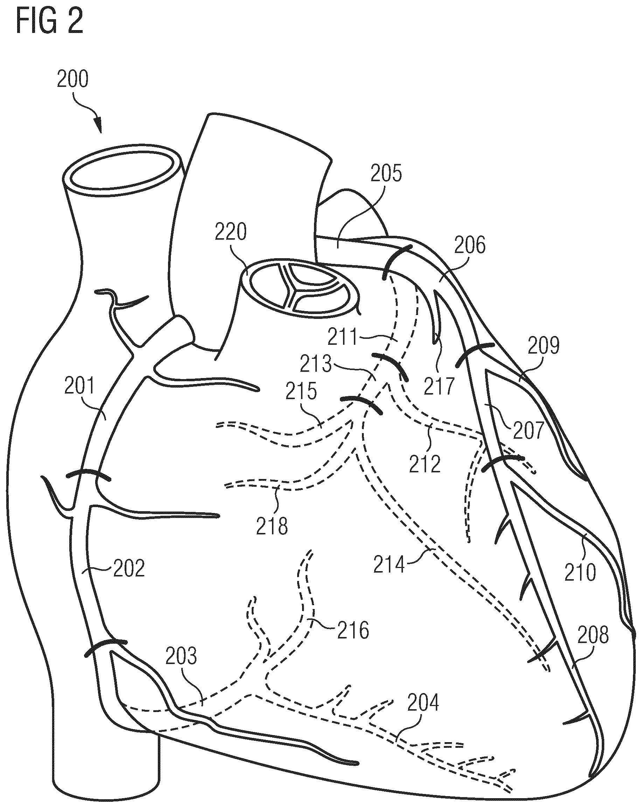

For example, the portion of coronary arteries within the anatomical region of interest may be one or more segments of the coronary arteries of the heart. FIG. 2 schematically illustrates an exemplary 3D frontal view 200 of a heart and of coronary arteries. The segments of the coronary arteries in FIG. 2 are based on and numbered according to the vessel segmentation as proposed by the American Heart Association (AHA) and as amended by the Society of Cardiovascular Computed Tomography (SCCT). Table 1 provides a short description of the respective vessel segments as well as an abbreviation for each vessel segment.

**Table 1: Segments of the Coronary Arteries in FIG. 2.**

| **Ref. Sign** | **Vessel Segment** | **Abbreviation** | **Description** |
|---|---|---|---|
| 201 | proximal right coronary artery | pRCA | Ostium of the RCA to one-half the distance to the acute margin of heart |
| 202 | Mid RCA | mRCA | End of pRCA to the acute margin of heart |
| 203 | Distal RCA | dRCA | End of mRCA to origin of the PDA (posterior descending artery) |
| 204 | PDA-R | R-PDA | PDA from RCA |
| 205 | Left main | LM | Ostium of LM to bifurcation of LAD (left anterior descending artery) and LCx (left circumflex artery) |
| 206 | Proximal LAD | pLAD | End of LM to the first large septal or D1 (first diagonal), whichever is most proximal |
| 207 | Mid LAD | mLAD | End of proximal LAD to one-half the distance to the apex |
| 208 | Distal LAD | dLAD | End of mid LAD to end of LAD |
| 209 | D1 | D1 | First diagonal branch D1 |
| 210 | D2 | D2 | Second diagonal branch D2 |
| 211 | Proximal LCx | pCx | End of LM to the origin of the OM1 (first obtuse marginal) |
| 212 | OM1 | OM1 | First OM1 traversing the lateral wall of the left ventricle |
| 213 | Mid and distal LCx | LCx | Traveling in the atrioventricular groove, distal to the OM1 branch to the end of the vessel or origin of the L-PDA |
| 214 | OM2 | OM2 | Second marginal OM2 |
| 215 | PDA-L | L-PDA | PDA from LCx |
| 216 | PLB-R | R-PLB | PLB from RCA |
| 217 | Ramus intermedius | RI | Vessel originating from the left main between the LAD and LCx in case of a trifurcation |
| 218 | PLB-L | L-PLB | PLB from LCx |

In addition to the vessel segments listed in Table 1, FIG. 2 also indicates the aortic valve 220.

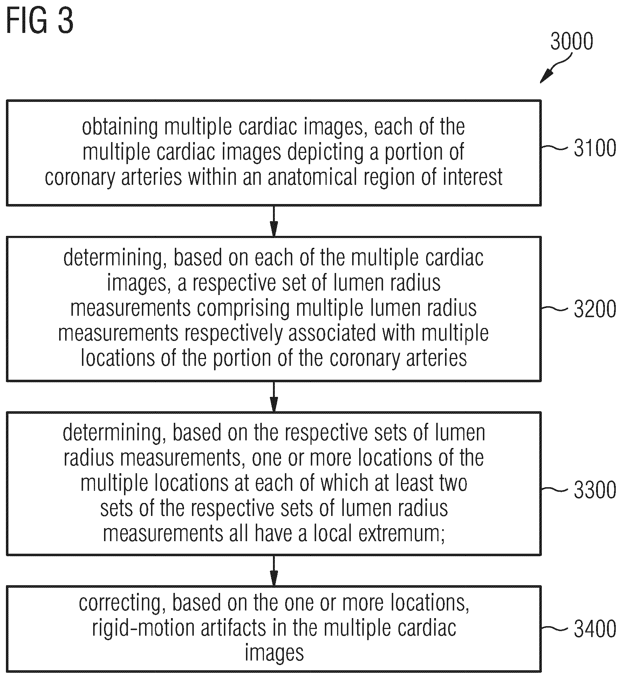

FIG. 3 illustrates aspects with respect to processing multiple cardiac images, each of which depicts a portion of coronary arteries, i.e., the same portion of coronary arteries, e.g., LAD (i.e., a combination of pLAD 206, mLAD 207, and dLAD 208), or RCA (i.e., a combination of pRCA 201, mRCA 202, and dRCA 203) as shown in FIG. 2, within an anatomical region of interest. The multiple cardiac images are obtained either during or after an angiography exam and thereby the processing can be either real-time processing or post-processing. A respective set of lumen radius measurements is determined based on each of the multiple cardiac images and comprises multiple lumen radius measurements respectively associated with multiple locations of the portion of the coronary arteries. One or more locations of the multiple locations, at each of which at least two sets of the respective sets of lumen radius measurements all have a local extremum, e.g., either a local maximum or a local minimum, are determined based on the respective sets of lumen radius measurements. One or more rigid-motion artifacts in the multiple cardiac images are corrected based on the one or more locations. Details of the method 3000 are described below.

Block 3100: obtaining multiple cardiac images, each of the multiple cardiac images depicting a portion of coronary arteries within an anatomical region of interest.

According to various examples, the multiple cardiac images may comprise cardiac images acquired under different acquisition angles.

For example, the multiple cardiac images may be obtained directly from an angiography device, e.g., the C-arm machine 800 of FIG. 1, under different acquisition angles. I.e., the multiple cardiac images may respectively depict different views of the same portion of coronary arteries. Additionally or optionally, the multiple cardiac images may be obtained under different acquisition angles and at the same time point of different cardiac cycles such as multiple successive cardiac cycles.

Alternatively, the multiple cardiac images may be obtained from a database for storing the multiple cardiac images acquired by the angiography device, e.g., a PACS.

Hereinafter, techniques will be explained using frames of X-ray angiograms as an example of cardiac images. I.e., a single cardiac image may correspond to a frame of an angiogram.

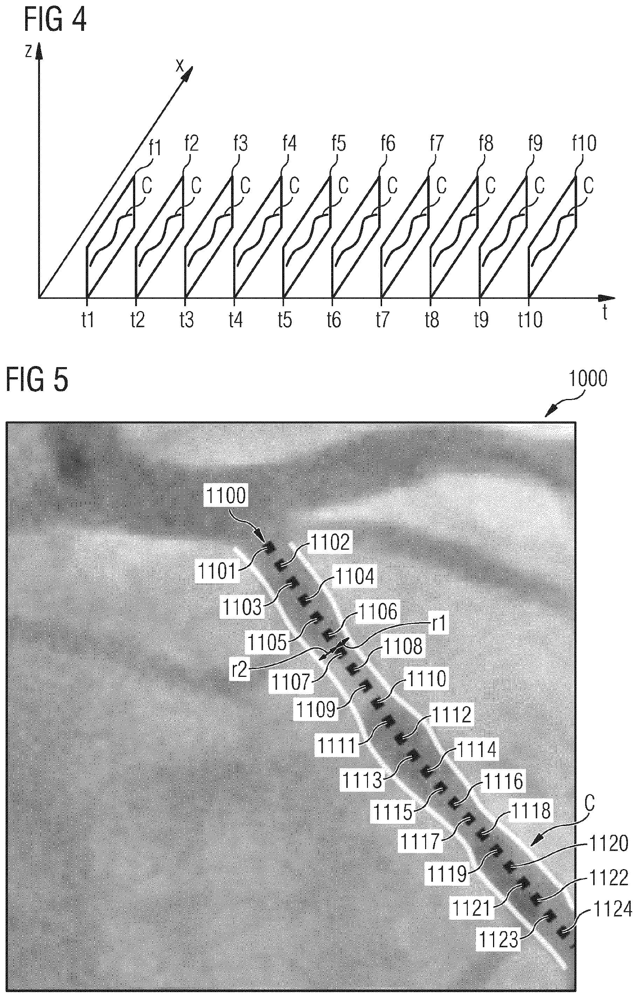

FIG. 4 schematically illustrates multiple frames f1-f10 of an angiogram acquired at different time points t1-t10 and each of the multiple frames f1-f10 depicts the same portion C of the coronary arteries within an anatomical region of interest.

For example, the multiple frames f1-f10 may be acquired during a single cardiac cycle of 1 second using a C-arm X-ray machine with a frame rate of 10. I.e., the multiple frames f1-f10 encapsulate information associated with different cardiac motions from multiple time points of a single cardiac cycle, e.g., respective states of the atria, the ventricles, and the valves. Such different cardiac motions may be associated with different blood pressures, aortic pressures, ventricular pressures, atrial pressures, ventricular volumes, and electrocardiograms.

To automatically and precisely correct rigid-motion artifacts in the multiple cardiac images, it is possible to automatically and precisely select or determine, among frames of an angiogram, multiple appropriate frames, where the entire portion C of the coronary arteries is visible.

According to various examples, said obtaining of the multiple cardiac images may comprise obtaining an angiogram acquired during an angiography exam of the anatomical region of interest and selecting, based on at least one pre-defined criterion, the multiple cardiac images among frames of the angiogram.

Such pre-defined criterion may comprise at least one of the following:
➢ the portion of coronary arteries is well visible;
➢ minimal foreshortening appears;
➢ no or minimal vessel overlaps appear;
➢ image quality, especially around the lumen borders, is good, e.g., above a threshold;
➢ less artifact presents; and
➢ the entire portion of coronary arteries is in the field of view.

According to various examples, the multiple cardiac images may be selected from frames acquired in different cardiac cycles but at the same time point of the respective cardiac cycles.

For example, an acquired angiogram may comprise 100 frames acquired in 10 cardiac cycles, e.g., first to tenth cardiac cycles, respectively under 10 different acquisition angles, and 10 frames, e.g., first to tenth frames, are acquired in each cardiac cycle under the same acquisition angle. A specific amount, e.g., 2, 3, 4, 5, or 6, of cardiac images may be respectively selected from frames acquired in the 10 different cardiac cycles. Each one of such cardiac images may be selected from respective frames acquired at the same time point, e.g., any one of t1 to t10 as shown in FIG. 4, within respective cardiac cycles.

According to various examples, after obtaining the multiple cardiac images, the method 3000 may further comprise determining the anatomical region of interest based on multiple predefined seed points.

For example, proximal seed points may comprise the catheter tip, and distal seed points may comprise one or more points selected on the main vessels or on side branches with significant stenoses.

Optionally, the portion of coronary arteries may be determined based on angulation information for acquiring the multiple cardiac images.

According to various examples, the method 3000 may further comprise segmenting the anatomical region of interest across the multiple cardiac images, i.e., separating the anatomical region of interest from the background in each of the multiple cardiac images.

Block 3200: determining, based on each of the multiple cardiac images, a respective set of lumen radius measurements comprising multiple lumen radius measurements respectively associated with multiple locations of the portion of the coronary arteries.

According to various examples, the lumen radius measurements may be determined or calculated using any one of the existing techniques or algorithms related to quantitative coronary angiography. For example, non-patent literature - Garrone, Paolo, et al. "Quantitative coronary angiography in the current era: principles and applications." Journal of interventional cardiology 22.6 (2009): 527-536.[1] - provides a comprehensive and updated viewpoint on quantitative coronary angiography. It is also conceivable that various existing machine learning algorithms or image processing techniques can be used to determine the lumen radius measurements.

FIG. 5 schematically illustrates one exemplary frame 1000 of an angiogram according to various examples. The frame 1000 may depict the portion C of coronary arteries as shown in FIG. 4. A centerline 1100 is generated between the starting point 1101 and the ending point 1124 of the portion C of coronary arteries. Between the starting point 1101 and the ending point 1124, there are multiple location points 1102-1123. For each of the location points 1101-1124, a respective lumen radius associated with such a location point may be measured or determined, and thereby a set of lumen radius measurements comprising the respective lumen radius at each of the location points 1101-1124 can be determined or calculated.

For example, at location point 1107, two lumen radii r1 and r2 may be determined or measured. The respective lumen radius associated with location point 1107 may be determined as an average of r1 and r2. It is also possible to determine or select the lumen radius associated with location point 1107 as either r1 or r2.

Similarly, for each one of the multiple frames f1-f10 of FIG. 4, a respective set of lumen radius measurements comprising the respective lumen radius at each of the location points 1101-1124 can be determined or calculated. I.e., ten sets s1-s10 of lumen radius measurements can be determined respectively for the ten frames f1-f10, and each of the ten sets s1-s10 comprises lumen radius measurements respectively associated with the location points 1101-1124.

To facilitate the determination of the respective sets s1-s10 of the lumen radius measurements, a segmentation algorithm may be applied to the ten frames f1-f10, respectively, to separate the portion C of the coronary arteries from the background of the respective frame.

According to various examples, said determining of the respective set of lumen radius measurements may comprise segmenting the portion C of the coronary arteries from the respective cardiac image of the multiple cardiac images and determining the respective set of the lumen radius measurements based on the respective segmented portion of the coronary arteries.

Block 3300: determining, based on the respective sets of lumen radius measurements, one or more locations of the multiple locations at each of which at least two sets of the respective sets of lumen radius measurements all have a local extremum, e.g., either a local maximum or a local minimum.

According to various examples, respective local extrema of the respective sets of lumen radius measurements may be determined based on second derivatives of respective curves, and each of the respective curves depicts a respective relationship between a respective set of the lumen radius measurements and the multiple locations of the portion of the coronary arteries.

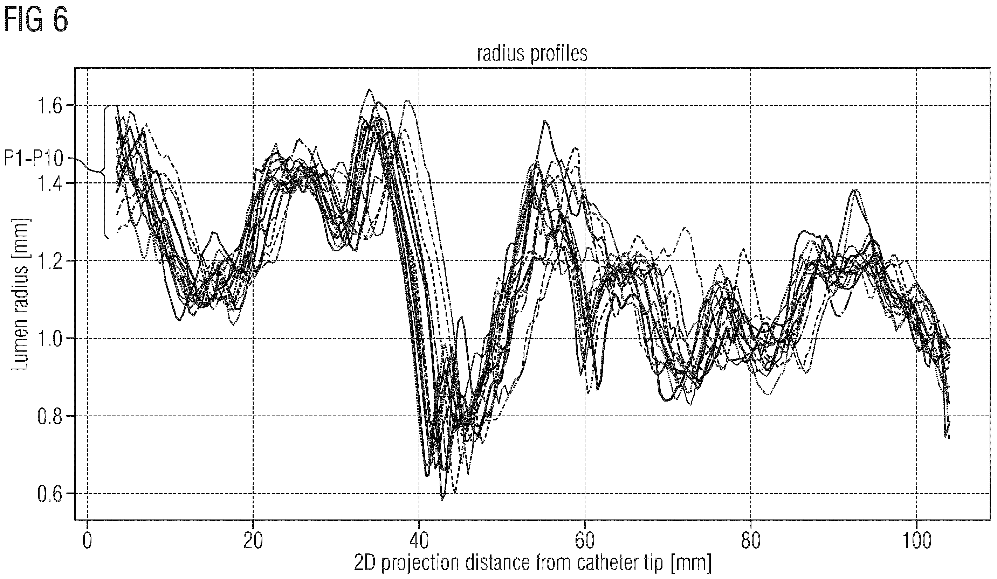

For example, as shown in FIG. 6, after determining the respective sets (e.g., 10 sets) of the lumen radius measurements, respective curves (or radius profiles) p1-p10 may be determined or plotted for each of the sets using interpolation such as linear interpolation, polynomial interpolation, spline interpolation, and/or mimetic interpolation. The x-axis of each curve may indicate respective distances (or 2D projection distances) between the catheter tip and each of the location points 1101-1124, and the y-axis may indicate respective lumen radii associated with the respective location points. Then, the respective local extrema of the respective sets of lumen radius measurements may be respectively determined based on local concavities or convexities of the respective curves p1-p10. For example, the second derivatives at each of the location points 1101-1124 may be calculated for each of the respective curves p1-p10. A respective point is a local maximum if the respective second derivative is less than 0, and a respective point is a local minimum if the respective second derivative is more than 0.

According to various examples, the second derivatives may be computed via central finite differences.

In practice, due to vessel deformations caused by heart motion, the respective sets of the lumen radius measurements (or the radius profiles/curves p1-p10) may be not aligned. To more precisely correct rigid-motion artifacts in multiple cardiac images, the respective sets of the lumen radius measurements (or the radius profiles/curves p1-p10) may be warped before determining the one or more locations of the multiple locations at each of which at least two sets of the respective sets of lumen radius measurements all have a local extremum.

For example, a dynamic time warping algorithm, e.g., as disclosed in a non-patent literature - Salvador, Stan, and Philip Chan. "Toward accurate dynamic time warping in linear time and space." Intelligent Data Analysis 11.5 (2007): 561-580.[2] - may be used for warping the respective sets of the lumen radius measurements. Alternatively, it is also possible to use an optimal subsequence bijection algorithm such as that disclosed in a non-patent literature - Latecki, Longin Jan, et al. "Optimal subsequence bijection." Seventh IEEE International Conference on Data Mining (ICDM 2007). IEEE, 2007.[3] It is still possible to use the techniques as disclosed in a further non-patent literature - Müller, Meinard. "Dynamic time warping." Information retrieval for music and motion (2007): 69-84.[4]

Optionally or additionally, the method 3000 may further comprise determining a reference set of the lumen radius measurements and aligning each of the respective sets of the lumen radius measurements with the reference set of the lumen radius measurements. Said determining of the one or more locations may be based on the respective aligned sets of the lumen radius measurements.

For example, the reference set of the lumen radius measurements may be randomly or arbitrarily selected from the respective sets of the lumen radius measurements. For instance, the first set is selected as the reference set of the lumen radius measurements and the other sets can be respectively aligned with the reference set, i.e., the first set, using dynamic time warping algorithm, e.g., the dtw function of Matlab, i.e., executing dtw (the first set, the second set), dtw (the first set, the third set), dtw (the first set, the fourth set), ..., dtw (the first set, the n-th set).

According to various examples, said determining of the reference set of the lumen radius measurements may comprise selecting the set of the lumen radius measurements having the longest length as the reference set of the lumen radius measurements. Optionally or additionally, the set of the lumen radius measurements having the longest length may be determined by sorting the respective sets of the lumen radius measurements based on respective lengths of the respective sets in descending or ascending order.

For example, each of the respective sets of the lumen radius measurements may be indicated as a row or column vector, and the length of a respective set corresponds to the number of elements or entries of the respective vector.

Optionally or additionally, said aligning may be based on one or more anatomical landmarks within the anatomical region of interest.

For example, the one or more anatomical landmarks may be associated with the portion of coronary arteries, e.g., bifurcations, stenoses, calcifications, and etc.

Block 3400: correcting, based on the one or more locations, rigid-motion artifacts in the multiple cardiac images.

For example, in order to correct the rigid-motion artifacts in the multiple cardiac images, the techniques as discloses in a non-patent literature - Banerjee, Abhirup, et al. "Point-cloud method for automated 3D coronary tree reconstruction from multiple non-simultaneous angiographic projections." IEEE transactions on medical imaging 39.4 (2019): 1278-1290.[5] - may be used. I.e., Rigid transformations may be estimated for all angiographic image acquisitions, which minimize the orthogonal distance between corresponding landmarks in object domain. The one or more locations determined at Block 3300 may be utilized as landmarks or matching landmarks.

Alternatively or additionally, it is also possible to employ the techniques disclosed in another non-patent literature - Yang, Jian, et al. "Novel approach for 3-D reconstruction of coronary arteries from two uncalibrated angiographic images." IEEE transactions on image processing 18.7 (2009): 1563-1572.[6]

According to various examples, rigid-motion artifacts in the multiple cardiac images may be corrected by optimizing a cost function iteratively as discloses in either document [5] or document [6]. The cost function may be formulated using a set of matching landmark points in multiple cardiac images, e.g., the one or more locations of the multiple locations at each of which at least two sets of the respective sets of lumen radius measurements all have a local extremum, e.g., either a local maximum or a local minimum.

According to various examples, the method 3000 may further comprise determining, based on the multiple cardiac images, at least one of a location of a catheter tip and one or more bifurcation locations of the portion of the coronary arteries. Said correcting of the rigid-motion artifacts may be further based on the location of the catheter tip and/or the one or more bifurcation locations.

According to various examples, additional camera parameters may be additionally or optionally considered as disclosed in document [6].

Alternatively or optionally, rigid-motion artifacts in the multiple cardiac images may be corrected by finding rigid motion between the multiple cardiac images using an iterative method disclosed in document [5]. The method disclosed in document [5] iterates between computing 3D locations of matching 2D points/landmarks and solving the absolute orientation problem as described in a non-patent literature - Horn, Berthold KP. "Closed-form solution of absolute orientation using unit quaternions." Josa a 4.4 (1987): 629-642.[7]

The method may comprise the following steps:
In the first step, 3D locations of matching 2D points are computed using midpoint method, i.e. 3D location is given by the point that is closest to the lines formed by matching points and the camera locations.
In the second step, rigid-motion-affected 3D points are found. These points are the projections of the 3D points from step 1 onto the line formed by the camera location of the second view and the 2D points on the second view. Absolute orientation between 3D points of the first step and the rigid-motion affected 3D points of the second step is the optimal rigid motion.
After the second step, the camera location and the 2D point locations are updated using the rigid transform. These two steps are iterated until convergence.

According to various examples, if the matching landmark points contain outliers (false matches), they can be processed using the techniques as disclosed in a non-patent literature - Fischler, Martin A., and Robert C. Bolles. "Random sample consensus: a paradigm for model fitting with applications to image analysis and automated cartography." Communications of the ACM 24.6 (1981): 381-395.[8] or another non-patent literature - Barath, Daniel, et al. "MAGSAC++, a fast, reliable and accurate robust estimator." Proceedings of the IEEE/CVF conference on computer vision and pattern recognition. 2020.[9] to solve the absolute orientation problem described in document [7] while eliminating the outliers.

According to this disclosure, the one or more locations of the multiple locations at each of which at least two sets of the respective sets of lumen radius measurements all have a local extremum can be used as matching 2D points or landmarks.

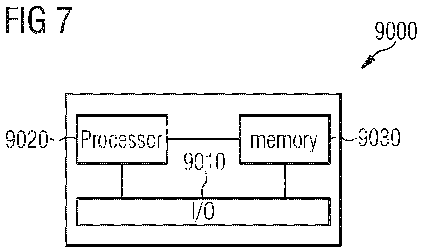

FIG. 7 is a block diagram of a computing device 9000 according to various examples. The computing device 9000 may comprise a processor 9020, a memory 9030, and an input/output interface 9010. The processor 9020 is configured to load program code from the memory 9030 and execute the program code. Upon executing the program code, the processor 9020 performs the method 3000 for processing multiple cardiac images depicting the same coronary artery of interest, e.g., the portion C of coronary arteries within an anatomical region of interest.

The computing device 9000 may be embedded in or connected with an angiography device such as the C-arm machine 800. I.e., the angiography device comprising the computing device 9000 may be configured to perform the method 3000.

Referring to FIG. 1 again, the C-arm machine 800 may further comprise the computing device 9000 configured to perform the method 3000. The computing device 9000 may be the C arm control unit 810 and/or the control panel 850. I.e., the computing device 9000 may be embedded in or connected with the C-arm machine 800, and thereby the C-arm machine 800 may be also configured to perform the method 3000.

Summarizing, techniques have been described that facilitate correcting rigid-motion artifacts in multiple cardiac images depicting a portion of coronary arteries within an anatomical region of interest. The techniques automatically determine one or more locations of the portion of the coronary arteries as landmarks or matching 2D points/landmarks for correcting one or more rigid-motion artifacts. The one or more locations are determined such that at least two sets of the respective sets of lumen radius measurements all have a local or relative extremum, e.g., either a local maximum or a local minimum, at such one or more locations. The techniques allow for a more precise 3D reconstruction of coronary arteries.

As a simple example, the techniques disclosed herein can be employed to automatically estimate the rigid motion between two non-simultaneous cardiac images, e.g., angiographic views, and thereby correct rigid-motion artifacts in the two cardiac images.

Although the disclosure has been shown and described with respect to certain preferred embodiments, equivalents and modifications will occur to others skilled in the art upon the reading and understanding of the specification. The present disclosure includes all such equivalents and modifications and is limited only by the scope of the appended claims.

For illustration, the disclosure is explained in detail based on X-ray angiography, the techniques disclosed herein can be also applied to angiography that may be done using scans instead of X-rays.

Further, the approaches described herein may be used also when certain parts of the region of interest are not well visible on a certain frame, e.g., due to foreshortening, vessel overlap, etc.

In addition, the approaches described herein may be used also when the multiple cardiac images are obtained from different angiography views / acquired under different acquisition angles. Certain regions of interest may be recommended depending on the angulation of the acquisition (e.g. LM/pLAD/proximal LCx for the spider view). By combining the recommended regions of interest from the various angulations, a more accurate representation of the entire region of interest may be obtained.

### List of Reference

[1] Garrone, Paolo, et al. "Quantitative coronary angiography in the current era: principles and applications." Journal of interventional cardiology 22.6 (2009): 527-536.
[2] Salvador, Stan, and Philip Chan. "Toward accurate dynamic time warping in linear time and space." Intelligent Data Analysis 11.5 (2007): 561-580.
[3] Latecki, Longin Jan, et al. "Optimal subsequence bijection." Seventh IEEE International Conference on Data Mining (ICDM 2007). IEEE, 2007.
[4] Müller, Meinard. "Dynamic time warping." Information retrieval for music and motion (2007): 69-84.
[5] Banerjee, Abhirup, et al. "Point-cloud method for automated 3D coronary tree reconstruction from multiple non-simultaneous angiographic projections." IEEE transactions on medical imaging 39.4 (2019): 1278-1290.
[6] Yang, Jian, et al. "Novel approach for 3-D reconstruction of coronary arteries from two uncalibrated angiographic images." leee transactions on image processing 18.7 (2009): 1563-1572.
[7] Horn, Berthold KP. "Closed-form solution of absolute orientation using unit quaternions." Josa a 4.4 (1987): 629-642.
[8] Fischler, Martin A., and Robert C. Bolles. "Random sample consensus: a paradigm for model fitting with applications to image analysis and automated cartography." Communications of the ACM 24.6 (1981): 381-395.
[9] Barath, Daniel, et al. "MAGSAC++, a fast, reliable and accurate robust estimator." Proceedings of the IEEE/CVF conference on computer vision and pattern recognition. 2020.

## Claims

1. A computer-implemented method (3000), comprising:
- obtaining (3100) multiple cardiac images, each of the multiple cardiac images depicting a portion of coronary arteries within an anatomical region of interest;
- determining (3200), based on each of the multiple cardiac images, a respective set of lumen radius measurements comprising multiple lumen radius measurements respectively associated with multiple locations of the portion of the coronary arteries;
- determining (3300), based on the respective sets of lumen radius measurements, one or more locations of the multiple locations at each of which at least two sets of the respective sets of lumen radius measurements all have a local extremum;
- correcting (3400), based on the one or more locations, rigid-motion artifacts in the multiple cardiac images.

2. The computer-implemented method (3000) of claim 1, further comprising:
- determining, based on the multiple cardiac images, at least one of a location of a catheter tip and one or more bifurcation locations of the portion of the coronary arteries;
wherein said correcting of the rigid-motion artifacts is further based on the location of the catheter tip and/or the one or more bifurcation locations.

3. The computer-implemented method (3000) of claim 1 or 2, wherein the multiple cardiac images are acquired during the same cardiac phase.

4. The computer-implemented method (3000) of any one of the preceding claims, wherein a count of the one or more locations is preferably 4 to 6.

5. The computer-implemented method (3000) of any one of the preceding claims, said obtaining of the multiple cardiac images comprising:
- obtaining an angiogram acquired during an angiography exam of the anatomical region of interest;
- selecting, based on at least one pre-defined criterion, the multiple cardiac images among frames of the angiogram.

6. The computer-implemented method (3000) of any one of the preceding claims, said determining of the respective set of lumen radius measurements comprising:
- segmenting the portion of the coronary arteries from the respective cardiac image of the multiple cardiac images; and,
- determining the respective set of the lumen radius measurements based on the respective segmented portion of the coronary arteries.

7. The computer-implemented method (3000) of any one of the preceding claims, further comprising:
- determining a reference set of the lumen radius measurements;
- aligning each of the respective sets of the lumen radius measurements with the reference set of the lumen radius measurements;
wherein said determining of the one or more locations are based on the respective aligned sets of the lumen radius measurements.

8. The computer-implemented method (3000) of claim 7, said determining of the reference set of the lumen radius measurements comprising:
- selecting the set of the lumen radius measurements having the longest length as the reference set of the lumen radius measurements.

9. The computer-implemented method (3000) of any one of the preceding claims, further comprising:
- determining the anatomical region of interest based on multiple predefined seed points.

10. The computer-implemented method (3000) of any one of the preceding claims, wherein the multiple cardiac images comprise cardiac images acquired under different acquisition angles.

11. A computing device (9000), the device comprising a processor (9020) and a memory (9030), wherein upon loading and executing program code from the memory (9030), the processor (9020) is configured to perform the method (3000) of any one of the preceding claims.

12. An angiography device (800) comprising the computing device (9000) of claim 11.

13. A program code executable by a processor, the processor, upon executing the program code, being configured to perform the method (3000) of any one of claims 1 to 10.
